# EUROPEAN PATENT APPLICATION

(11) **EP 2 944 253 A1**
(43) Date of publication of application: **18.11.2015**
(21) Application number: 13871235.1
(22) Date of filing: 09.01.2013
(51) Int. Cl.: A61B 5/053

(54) **METHOD AND APPARATUS FOR ACQUIRING SIGNALS FOR ELECTRICAL IMPEDANCE TOMOGRAPHY**

(71) Applicant: Timpel S.A., CEP-05417-020 São Paulo, SP (BR)
(72) Inventor: MAKTURA, Edward, Lazo, 04455-210 São Paulo - SP (BR); LIMA, Celso, Gonzalez, 22411-003 Rio de Janeiro - RJ (BR)
(74) Representative: Schwabe - Sandmair - Marx
(86) International application number: PCT/BR2013/000008
(87) International publication number: WO 2014/107772

(57) **Abstract**

The method of the invention comprises the stages of injecting an alternating signal AC (carrier) into at least one electrode positioned on the skin of the patient in a region of interest, measuring the signal induced into at least one electrode, extracting at least an envelope of the signal measured and determining the difference between said envelope and a reference signal. Said envelope may be extracted by demodulating the signal measured, and this demodulation may be carried out by way of an associated rectifier diode, in a preferred embodiment, and an RC filter. The reference signal may be an envelope corresponding to the demodulated signal of another electrode, the injected signal, earth or a fixed voltage value.

## Description

### PRIORITY CLAIM

This application is a national phase entry of International Patent Application PCT/BR2013/000008, filed January 9, 2013, designating the European Convention and published in Portuguese as International Patent Publication WO 2014/107772 A1 on July 17, 2014, the disclosure of which is hereby incorporated herein in its entirety by this reference.

### FIELD OF THE INVENTION

The present invention pertains to the field of electrical impedance tomography and, more particularly, to the treatment of signals measured through the electrodes positioned in contact with the patient's skin.

### BACKGROUND OF THE ART

Electrical impedance tomography is a known and widely used technique consisting of positioning a plurality of electrodes around a region of the patient's body, such as, for example, his chest, and injecting electrical excitation signals accompanied by measuring the other electrodes of the induced signals so as to generate a map indicative of impedance, whereby it is possible to determine respiratory and hemodynamic parameters of the patient, and to generate images that represent these parameters.

In each measurement cycle the excitation electrodes are sequenced so as to include the majority or all the electrodes installed around the region of interest, systems with 16 or 32 electrodes being typically used. Generally, 20 to 50 measurement cycles per second (images per second) are generated, and alternating excitation signals with a frequency typically between 10 kHz and 2.5 MHz are used. These characteristics enable Electrical Impedance Tomography apparatuses to capture well the main characteristics of hemodynamics and breathing of human patients, which are phenomena with a frequency typically of the order of 1 to 4 Hz in the case of heartbeats and lower than 1 Hz in breathing.

According to known techniques, an alternating electrical signal is injected between at least two electrodes, also inducing alternating signals which are measured in the other electrodes. Through the differences between the injected signal and the signals measured, and between the very signals measured, followed by digital signal processing techniques, a map representative of the impedances of the region of interest is constructed. It is fundamental to note that this technique presupposes that the induced alternating signals which were measured in the electrodes are synchronized with each other and also with the injected signal, which rarely occurs since the materials traversed by each of these signals do not have a uniform composition, and may present impedance variations both in the resistive component and in the reactive component. Accordingly, the phases of the signals measured should be offset by the equipment for the construction of impedance maps.

Given the fact that each measurement cycle comprises a plurality of stages, these differing in that said injection is made in different electrodes at each stage, the equipment must continuously adjust the phases of the signals measured during the course of said cycle, meaning Electrical Impedance Tomography apparatuses are required to be significantly complex, both from the perspective of circuit tuning and in harmony with processing capacitance.

Moreover, the adjustment values of the phases depend on the frequency of the injected signal, making this adjustment complex when using more than one frequency to generate the impedance maps.

### OBJECTIVES OF THE INVENTION

In view of the above, the objective of the present invention is to provide a method and an apparatus for carrying out electrical impedance tomographies, by injecting a high frequency alternating current into successive pairs of electrodes, measuring the induced voltages in the other electrodes and extracting low frequency signals indicative of hemodynamic and breathing activities.

Another objective consists of providing a method of extracting low frequency signals that dispenses with the phase adjustment inherent in traditional methods of Electrical Impedance Tomography, which currently presupposes synchronization between the induced and injected signals.

Another objective consists of providing a method and apparatus in which the change of frequency of the injected signal does not imply adjustments in the extraction device of low frequency signals.

### BRIEF DESCRIPTION OF THE INVENTION

The above objectives, as well as others, are achieved by the invention through a method that comprises the following stages:
- Injecting an alternating AC signal (carrier), through electrodes positioned around the region of interest of a patient's body;
- Measuring the induced AC signal in at least one electrode;
- Demodulation of said AC signal measured for extracting at least an envelope, by means of rectification and optional filtering;
- Identifying the difference between the demodulated signal and a reference signal, so as to provide data for determining the impedance map corresponding to the region of interest.

According to another characteristic of the invention, after said rectification at least an envelope of the AC signal measured is extracted.

According to another characteristic of the invention, said extraction of the envelope is provided by filtering said demodulated signal.

According to another characteristic of the invention, the method additionally comprises identifying the difference between said envelope and a reference signal.

According to another characteristic of the invention, the reference signal is the signal measured in another electrode.

According to another characteristic of the invention, the reference signal is the injected signal.

According to another characteristic of the invention, the reference signal is earth or a fixed voltage value.

According to another characteristic of the invention, the frequency of the carrier may be varied depending on the anatomical characteristics of the patient.

According to another characteristic of the invention, the signal is injected into one electrode or simultaneously into more than one electrode.

According to another characteristic of the invention, the signal is injected successively into electrodes positioned on the body so as to complete a measurement cycle around the region of interest.

According to another characteristic of the invention, at least one electrode is not used for injecting the signal so as to complete the measurement cycle around the region of interest.

According to another characteristic of the invention, the difference between said envelope and the reference signal is provided by subtracting the signals through an analogical electronic circuit.

According to another characteristic of the invention, the difference between said envelope and the reference signal is provided by subtracting the signals through digital signal processing.

According to another characteristic of the invention, the measurements of the induced signals in the electrodes occur simultaneously.

According to another characteristic of the invention, the apparatus for acquiring electrical impedance tomography signals comprises at least a current source, at least a demodulation circuit per channel, at least a circuit for analogical subtraction between the demodulated signal and a reference signal and at least a circuit for conversion from analogical to digital (A/D) from that resulting from the subtraction between the demodulated signal and a reference signal.

According to another characteristic of the invention, the current source generating the injection signal is of the *single ended* type.

According to another characteristic of the invention, the current source generating the injection signal is if the bipolar type.

According to another characteristic of the invention, each electrode has a corresponding current source.

According to another characteristic of the invention, the circuit's fixed capacitances are offset by at least a Negative Impedance Converter (NIC) circuit.

According to another characteristic of the invention, said fixed capacitances are the input capacitances of the operational amplifier.

According to another characteristic of the invention, said fixed capacitances are analog switch capacitances.

According to another characteristic of the invention, the demodulation circuit comprises a rectifier diode.

According to another characteristic of the invention, the demodulation circuit comprises a rectifier diode and an RC filter.

According to another characteristic of the invention, the demodulation circuit provides an *offset* for the rectifier diode, be it positive or negative offset.

According to another characteristic of the invention, each channel receives signals corresponding to more than one electrode.

### DESCRIPTION OF THE DRAWINGS

Other characteristics and advantages of the present invention will become more obvious in the description of a preferred embodiment, given as an example as opposed to imposing limits, and of the drawings referring thereto, wherein:
Figure 1 schematically illustrates the positioning of the electrodes on the surface of a region of interest of a patient.
Figure 2 illustrates the circuit that treats the signals captured by two electrodes.
Figures 3 to 8 illustrate the signal wave forms, corresponding to various stages of the treatment of the signal captured by two electrodes.
Figure 9 exemplifies a circuit endowed with additional characteristics, to carry out the processing of the signal captured by one of the electrodes.

### DETAILED DESCRIPTION OF THE INVENTION

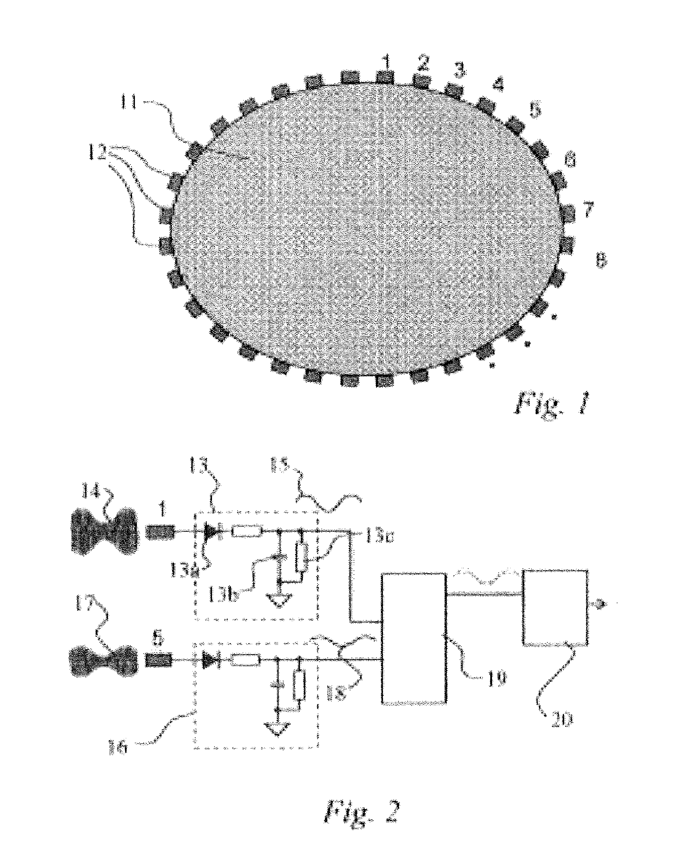

Figure 1 schematically illustrates the application of a plurality of electrodes 12 around a region of interest of a patient 11, according to known techniques. The present example uses 32 electrodes, referenced in sequence with the numbers 1, 2, 3, etc., it being understood that the invention is applicable to a different number of electrodes, which may be distributed uniformly or otherwise.

According to known principles expounded in prior documents, such as, for example, US4617939, US5311878, US5626146, US5807251 and US5919142, an excitation current is injected into at least one of the electrodes and then the voltages induced into the other electrodes are measured. In the most commonly used prior embodiments, a current is applied between a pair of adjacent electrodes and the voltage between the other pairs of adjacent electrodes is measured.

Even though acceptable results have been obtained with the technique referred to above, arrangements can be used in which the excitation current is applied to non-adjacent electrodes, one, two or more electrodes being spaced between the excitation electrodes, and the same may occur with the measurement electrodes. Said arrangements are designated "interleave."

For interleave three, for example, the apparatus injects an alternating AC current into electrodes 1 and 5 and takes the simultaneous measurement of the induced signals in the other pairs of electrodes (2-6; 3-7; 4-8; 6-9;7-10 ...).

Next, it injects an AC current into electrodes 2 and 6 and again takes the **simultaneous** measurement of the induced signals in the other pairs of electrodes (1-5; 3-7; 4-8; 6-9;7-10 ...), and so on and so forth, until completing a measurement cycle around the region of interest.

Figure 2 schematically shows the elements of the circuit related to the treatment of the signals measured by electrodes 1 and 5. As illustrated, the alternating signal 14 measured by the electrode 1 is introduced into the set of components 13, which comprises a rectifier diode 13a and the RC filter comprising the capacitor 13b and the resistor 13c. The output of the set 13 is an envelope 15 of the original signal, which in the present example is the upper envelope. The signal 17 captured by the electrode 5 is subjected to identical treatment by the set 16, resulting the signal 18, which is the envelope superior of the originally measured signal 17. This signal is used as reference in the following signal treatment stage, performed by the instrumentation amplifier (with or without programmable gain) 19, this stage consisting of identifying the difference between signals 15 and 18, obtained, for example, subtracting the reference signal 18 from the signal 15. The result of this subtraction is then sent to the converter A-D 20, the digitalized output being sent for processing by a controller (not illustrated).

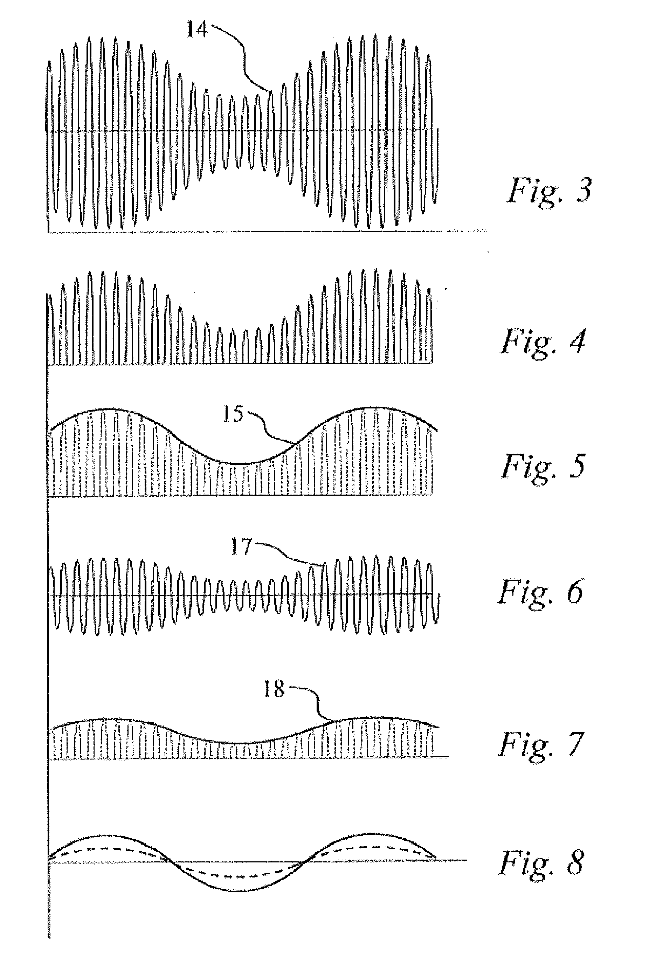

Figures 3 to 8 illustrate the signals corresponding to the various phases of the proposed method. Figure 3 illustrates the wave (carrier) in high frequency 14 (over 10 kHz, preferably 75 kHz or above) measured by electrode 1. After rectifying the half wave, the semi-senoids illustrated in Fig. 4 are obtained, and after filtering, these supply an envelope 15 as per Fig. 5. Figure 6 illustrates the high frequency wave 17 measured by electrode 5, which, after rectification and filtering, results in the envelope 18 seen in Fig. 7. Figure 8 illustrates the overlapping of said envelopes, each line corresponding to the envelope 15 and the dotted line to the envelope 18. An envelope 15 and an envelope 18, which in this case corresponds to the reference signal, are input to the block 19, whose output is a resulting signal corresponding to the difference between the input signals 15 and 18. It is worth noting that the envelopes 15 and 18 extracted from the signals 14 and 17 are in the illustrated case upper envelopes, and may alternatively be lower envelopes or even a combination of upper and lower envelopes.

Further, it is emphasized that in the proposed method there is no need for any offsetting of a potential phase difference between the high frequency signals measured by electrodes 1 and 5, since the signals of interest are the envelopes 15 and 18, which are not affected, or minimally affected by said phase difference. Furthermore, bearing in mind that the signals of interest are the envelopes and not the carrier, the frequency thereof may be adapted to the patient's conditions or physical constitution so as to optimize the system operation.

The signal injected in the patient can be generated by means of known circuits, such as a single ended or bipolar current source. Furthermore, a single source may be used for the entire system, or individual sources for each electrode, in which case the switching elements between the source and each electrode will be dispensed with.

In the diagram in Fig. 2, each signal treatment channel signal 13 or 16 receives the signal from a single electrode, respectively 1 or 5. However, each channel may receive the signal of more than one electrode, the additional signal being the one coming from a reference electrode, the subtraction between the envelopes being carried out in the channel itself.

Further in connection with the rectification and filtering circuit, an auxiliary circuit may be provided to produce an *offset* in the diode 13 a, by applying a continuous positive or negative pre-polarization voltage so as to make the diode operates in the active region (in the case of positive pre-polarization), enabling the rectification of low amplitude signals.

Signal demodulation can therefore be understood as the process of extracting the envelopes (e.g.: 15 and 18) from the signals (e.g.: 14 and 17) measured in the electrodes (e.g.: 1 and 5). It consists, for example, of rectifying the signal measured optionally followed by filtering.

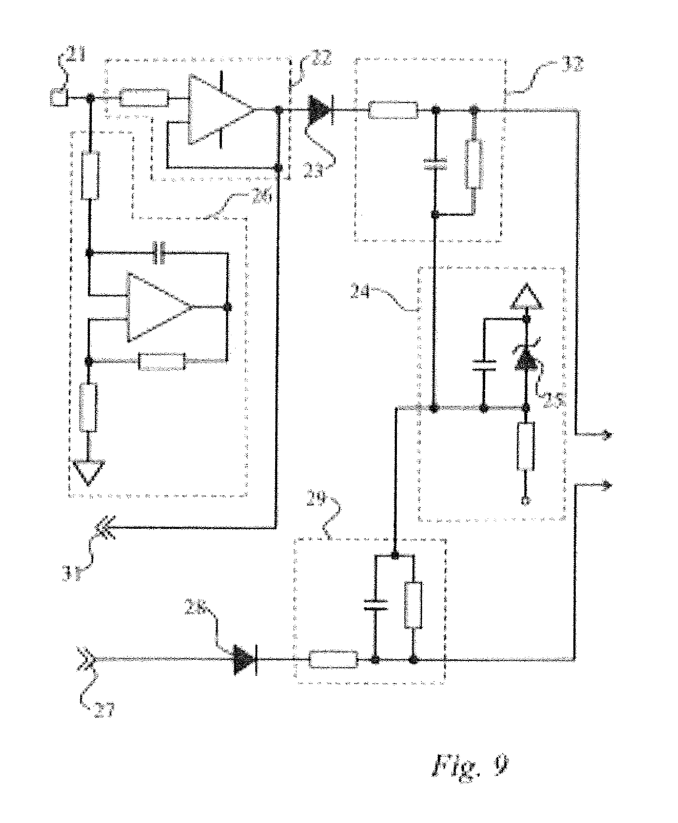

In an alternative or optional form of the invention, the fixed capacitances of the input circuit may be offset by using a Negative Impedance Converter - NIC. These may be input capacitances of the operational amplifier or those associated to the analog switch. Figure 9 exemplifies the use of a circuit endowed with a NIC 26 stage, associated to the input circuit 22, a *buffer* with high input impedance for processing the signal measured by the electrode 21 in electrical contact with the patient. Said input buffer 22 supplies the signal that will be rectified by the diode 23, as well as a signal, present in the terminal 31, that may be used as reference signal for another channel. This drawing also shows the presence of a pre-polarization circuit 24 of the diode 23, which introduces an *offset* determined by the voltage of the reference diode 25. The terminal 27 is the point of introduction of a reference signal already bufferized, which will be rectified by the diode 28 and filtered by the subset 29, so as to supply a low frequency signal which will be sent to the subtraction circuit (not illustrated) jointly with the low frequency signal coming from the filter 32, which corresponds to an envelope of the high frequency signal captured by the electrode 21.

## Claims

1. Method for acquiring signals for electrical impedance tomography, **characterized by** comprising the following basic stages:
a. injecting an alternating signal AC (carrier) into at least one electrode positioned in a region of interest;
b. measuring the signal induced in at least one electrode;
c. extracting at least an envelope from the signal measured;
d. identifying the difference between said envelope and a reference signal.

2. Method as claimed in claim 1, characterized wherein the envelope is extracted by signal demodulation obtained by said measure in at least one electrode.

3. Method as claimed in claims 1 or 2, characterized wherein the reference signal is an envelope corresponding to the demodulated signal of another electrode.

4. Method as claimed in claim 1, characterized wherein the reference signal is the injected signal.

5. Method as claimed in claim 1, characterized wherein the reference signal is the earth.

6. Method as claimed in claim 1, characterized wherein the reference signal is a fixed voltage value.

7. Method as claimed in claim 1, characterized wherein the injected signal AC (carrier) may vary in frequency.

8. Method as claimed in claim 1, characterized wherein the signal is injected into more than one electrode at the same time.

9. Method as claimed in claim 1, characterized wherein the signal is injected successively into electrodes positioned on the body so as to complete a measurement cycle around the region of interest.

10. Method as claimed in claims 1 or 9, characterized successively at least one electrode is not used for injecting the signal so as to complete the measurement cycle around the region of interest.

11. Method as claimed in claim 1, characterized wherein the difference between said envelope and the reference signal is provided by subtracting the signals through an electronic analog circuit.

12. Method as claimed in claim 1, characterized wherein the difference between said envelope and the reference signal is provided by subtracting the signals through digital signal processing.

13. Method as claimed in claim 1, characterized wherein the signals induced in the electrodes are measured simultaneously.

14. Apparatus for acquiring electrical impedance tomography signals according to the method defined by any of claims 1 to 13, **characterized by** comprising:
a. at least a current source;
b. at least a demodulation circuit per channel;
c. at least a circuit for analog subtraction between the demodulated signal and a reference signal;
d. at least a circuit for converting analog to digital (A/D) from that resulting from the subtraction between the demodulated signal and a reference signal.

15. Apparatus as claimed in claim 14, characterized wherein the current source is of the single ended type.

16. Apparatus as claimed in claim 14, characterized wherein the current source is of the bipolar type.

17. Apparatus as claimed in claim 14, characterized wherein each electrode has a corresponding current source.

18. Apparatus as claimed in claim 14, characterized wherein the fixed capacitances of the circuit are offset by at least one Negative Impedance Converter - NIC circuit.

19. Apparatus as claimed in claim 18, characterized wherein said fixed capacitances are the input capacitances of the operational amplifier.

20. Apparatus as claimed in claim 18, characterized wherein said fixed capacitances are analog switch capacitances.

21. Apparatus as claimed in claim 14, characterized wherein the demodulation circuit comprises a rectifier diode.

22. Apparatus as claimed in claim 21, characterized wherein the demodulation circuit comprises a rectifier diode and an RC filter.

23. Apparatus as claimed in claims 14, 21 or 22, characterized wherein the demodulation circuit provides an *offset* for the rectifier diode.

24. Apparatus as claimed in claim 23, characterized wherein the *offset* is positive.

25. Apparatus as claimed in claim 23, characterized wherein the *offset* is negative.

26. Apparatus as claimed in claim 14, characterized wherein the circuit of each channel receives signals corresponding to more than one electrode.
